# EUROPÄISCHE PATENTSCHRIFT

(11) **EP 1 831 163 B1**
(45) Veröffentlichungstag und Bekanntmachung des Hinweises auf die Patenterteilung: **26.09.2012**
(21) Anmeldenummer: 05821674.8
(22) Anmeldetag: 17.12.2005
(51) Int. Cl.: C07D 207/12

(54) **VERFAHREN ZUR HERSTELLUNG VON PYRROLIDONEN AUS SUCCINATEN AUS FERMENTATIONSBRÜHEN**
METHOD FOR PRODUCING PYRROLIDONES FROM SUCCINATES FROM FERMENTATION BROTHS
PROCEDE POUR PRODUIRE DES PYRROLIDONES A PARTIR DE SUCCINATES ISSUS DE BOUILLONS DE FERMENTATION

(30) Priorität: 21.12.2004 DE 102004062717
(43) Veröffentlichungstag der Anmeldung: 12.09.2007
(73) Patentinhaber: BASF SE, 67056 Ludwigshafen (DE)
(72) Erfinder: FISCHER, Wolfgang, 67360 Lingenfeld (DE); KLEIN, Daniela, 68161 Mannheim (DE); KÜNKEL, Andreas, 67434 Neustadt (DE); PINKOS, Rolf, 67098 Bad Dürkheim (DE); SCHOLTEN, Edzard, 68159 Mannheim (DE)
(86) Internationale Anmeldenummer: PCT/EP2005/013630
(87) Internationale Veröffentlichungsnummer: WO 2006/066839

(56) Entgegenhaltungen:
- EP-A- 0 410 728
- US-A- 4 841 069

## Beschreibung

Bernsteinsäure und deren Derivate sind wichtige Vorprodukte für eine Reihe von wirtschaftlich interessanten Produkten und finden z.B. Verwendung bei der Herstellung von Alkyd- und Polyester-Harzen, Lösungsmitteln oder einer Reihe von Spezialchemikalien, s. Zeikus, Appl. Microbiol. Biotechnol, 1999, 545-552. Pyrrolidone, die man auch zuweilen Pyrrolidinone nennt, werden im wesentlichen petrochemisch aus der Reaktion von Gamma-Butyrolacton mit Ammoniak (2-Pyrrolidon) bzw. mit Methylamin (N-Methylpyrrolidon) oder allgemein mit Alkylamin (N-Alkylpyrrolidon) hergestellt. N-Alkylpyrrolidone, wie z.B. NMP, finden z.B. in der Elektroindustrie als Lösemittel Verwendung, 2-Pyrrolidon ist ein wichtiges Lösungs- und Extraktionsmittel und dient überwiegend als Zwischenprodukt zur Herstellung von N-Vinylpyrrolidon, das wiederum ein wichtiges Monomer darstellt.

Bisher existiert nur ein relativ kleiner Markt für Bernsteinsäure, die in der Regel petrochemisch aus Maleinsäureanhydrid oder Butandiol hergestellt wird, jedoch wurde in den letzten Jahren sehr stark auf dem Gebiet der fermentativen Herstellung geforscht, so dass eine industrielle fermentative Herstellung von Bernsteinsäure als Zwischenprodukt für C4-Chemikalien in naher Zukunft möglich erscheint; Miller, Varadarajan in Biotechnol. Prog. 1999,15,845-854. Aufreinigungsmethoden zur Isolierung von Bernsteinsäure oder Bernsteinsäuresalzen aus Fermentationsbrühen sind zahlreich beschrieben. Bernsteinsäure wird beispielsweise mit Hilfe von Filtration, Kristallisation, Extraktion, Elektrodialyse, Chromatographie, aus Fermentationsbrühen gewonnen: US 5,034,105, US 4,670,155, US 5,814,498, US 5,168,055, US 5,958,744, US 5,780,276, US 5,143,834, US 5,143,833, US 5,412,126, US 5,104,492, US 4,670,155.

Die aufgereinigte Bernsteinsäure und ihre Salze oder deren Ester werden in der Regel zu N-Alkylsuccinimid (US 4,841,069, US 4,814,464) oder direkt zu 2-Pyrrolidon (US 3,080,377, US 3,198,808, US 3,681,387, US 4,263,175) oder NMP (US 3,448,118, US 5,157,127, US 5,434,273, WO02/102772) umgesetzt.

Weiterhin werden Verfahren beschrieben, in denen Pyrrolidone aus Succinimid hergestellt werden. Succinimid selbst kann nach JP 04282361 aus Bernsteinsäure und Ammoniak in Gegenwart von Phopsphit hergestellt werden. SU 1317890 beschreibt die Herstellung von Succinimid aus Bernsteinsäure und Harnstoff, während gemäß Zh. Obshch. Khim.; 20, 1950, 1145, 1149, 1191, 1195 Succinimid zusammen mit Bernsteinsäuremonoamid aus Bernsteinsäure in Gegenwart von Pyridin und Sulfamid hergestellt werden kann. Weitere Verfahren zur Herstellung von Succinimid sind z.B. beschrieben in Yakugaku Zasshi; 62; 1942; 532, 169, wo Succinimid aus Bernsteinsäure mit Formamid hergestellt wird, Recl. Trav. Chim. Pays-Bas; 75; 1956; 164, 167; J. Indian Chem. Soc.; 10; 1933; 111, 114, die die Herstellung von Succinimid aus Bernsteinsäure und Harnstoff beschreiben sowie in den Veröffentlichungen J. Chem. Soc.; 1931; 443 (Herstellung von Succinimid aus Bernsteinsäure und Ammoniumcarbonat), Chem. Ber.; 23; 1890; 3285 (Herstellung von Succinimid aus Bemsteinsäuremonoamid) und Justus Liebigs Ann. Chem.; 49; 1844; 197 (Herstellung von Succinimid aus Bemsteinsäurediamid).

In Clarke, Org. Synth. 1943, II, 562 wird die Umsetzung von chemisch reinem Diammoniumsuccinat zu Succinimid durch eine Reaktivdestillation mit anschließender Destillation beschrieben. In einer ersten Stufe wird die wässrige Diammoniumsuccinatlösung eingeengt bis eine Schmelze vorliegt und dann in einer zweiten Stufe wird das Succinimid über Kopf abdestilliert. Beispielsweise wird eine Kristallisation des Succinimids in 95 %-igem Ethanol durchgeführt, um eine möglichst hohe Reinheit zu erreichen.

DT 2313386 beschreibt die chemische Umsetzung von Bernsteinsäure in Gegenwart von Ammoniak und Triammoniumphosphat zu Succinimid.

WO 02/102772 beschreibt ein Verfahren zur Herstellung von N-Methylpyrrolidonen, bei dem Diammoniumsuccinat hydriert wird und ein Gemisch aus 2-Pyrrolidon und N-Methylpyrrolidon entsteht.

Bei der Herstellung von Folgeprodukten, wie z. B. Pyrrolidonen, aus fermentativ hergestellter Bernsteinsäure und ihren Derivaten erfordert die erfolgreiche Durchführung der Reaktionsschritte oftmals, dass die Intermediate aufgereinigt werden müssen. Zur Aufarbeitung von Bernsteinsäure oder ihren Derivaten (z. B. Salze oder Ester) aus Fermentationsbrühen werden oftmals apparativ und zeitlich aufwändige Verfahrensschritte wie z. B. Kristallisation, Fällung, Elektrodialyse, Extraktion oder Chromatographie vorgeschlagen, um eine ausreichende Abtrennung der z. B. katalysatorschädigenden Inhaltsstoffe (evtl. Nährmedienbestandteile, Stoffwechselnebenprodukte, Zellbestandteile) zu erreichen. Da insbesondere für Bernsteinsäure und ihre Salze oftmals eine Kombination dieser Verfahrensschritte als notwendig beschrieben wird, waren die dadurch entstehenden Kosten bisher ein Hemmnis für ein industrielles Verfahren zur Umsetzung von fermentativer Bernsteinsäure zu vergleichsweise niedrigpreisigen Produkten wie z. B. Pyrrolidonen. Zudem ist bei einigen dieser Aufarbeitungsverfahren ein Recycling der in der Fermentation eingesetzten Base nicht möglich, was zusätzliche Kosten hinsichtlich Einsatzstoffe und Entsorgung verursacht.

Der vorliegenden Erfindung lag also das Problem zugrunde, ein Verfahren zur Verfügung zu stellen, das eine kostengünstige Herstellung von Succinimid sowie möglichen Folgeprodukten, insbesondere von substituierten und nicht-substituierten Pyrrolidonen aus verunreinigten Succinat-haltigen Lösungen, d.h. aus Lösungen, die viele Nebenkomponenten enthalten, wie z.B. Fermentationsbrühen, erlaubt.

Das dieser Erfindung zugrunde liegende Problem wird durch das Zurverfügungstellen des hierin beschriebenen erfindungsgemäßen Verfahrens und die in den Ansprüchen charakterisierten Ausführungsformen gelöst.

Folglich betrifft die vorliegende Erfindung ein Verfahren zur Herstellung von dem Zwischenprodukt Succinimid oder einer Zusammensetzung, die Succinimid enthält, und in welchem in weiteren erfindungsgemäßen Schritten Pyrrolidone hergestellt werden können. Das erfindungsgemäße Verfahren wird durch die hierin beschriebenen Ausführungsformen charakterisiert.

In einer Ausführungsform betrifft die vorliegende Erfindung folglich ein Verfahren zur Herstellung der Verbindung II oder einer Zusammensetzung, enthaltend die Verbindung II 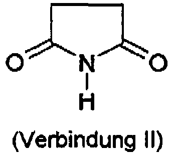 wobei das Verfahren mindestens folgende Schritte umfasst:
(a) Zurverfügungstellen einer Fermentationsbrühe, die in einer weiteren Ausführungsform vorbehandelt ist, z.B. durch Biomasseabtrennung, Sterilisation, und/oder Aufkonzentrierung, enthaltend die Verbindung I 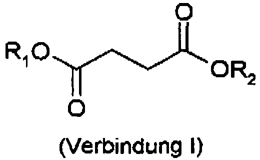 wobei R₁ gleich NH₄⁺, H⁺ oder ein anderes Kation und
   R₂ gleich NH₄⁺, H⁺ oder ein anderes Kation sein kann,
   und wobei mindestes R₁ oder R₂ gleich NH₄⁺ ist;
(b) Umsetzen der Verbindung I in der Fermentationsbrühe zur Verbindung II;
(c) gleichzeitiges, intermittierendes oder folgendes Abdestillieren von Ammoniak und/oder Wasser;
(d) nachfolgendes Destillieren des Sumpfes der Destillation von Schritt (c) bei vermindertem Druck, wobei ein Destillat enthaltend Verbindung II entsteht; und
(e) Isolieren der Verbindung II oder Umsetzen des Destillats aus Schritt (d).

In Schritt (c) wird Wasser teilweise oder vollständig abdestilliert, das in der Reaktion entsteht oder aber in der Brühe enthalten ist. Ammoniak entsteht nur, wenn R₁ und R₂ für Ammonium stehen. Zusätzlich können noch andere flüchtige Stoffe, vor allem Stoffe die leichter als Succinimid sind mit abdestilliert werden, wie z. B. Ethanol, das bei der Fermentation als Nebenprodukt entstehen kann. Bei den Kationen handelt es sich im wesentlichen um Kationen, die überlicherweise in Fermentationsbrühen oder deren Aufbereitungen enthalten sind. In einer weiteren Ausführungsform ist das Kation ein einwertiges Kation, beispielsweise eines der folgenden Kationen: Na+ und/oder K+. In einer weiteren Ausführungsform ist das Kation ein zweiwertiges Kation, beispielsweiseMg²⁺, und/oder Ca²⁺. In einer weiteren Ausführungsform liegt eine Mischung verschiedener Kationen vor, beispielsweise Na+ und/oder K+. In einer weiteren Ausführungsform ist das Kation ein zweiwertiges Kation, beispielsweiseMg²⁺, und/oder Ca²⁺ und/oder andere Kationen, beispielsweise von Spurenelementen.

Überraschenderweise wird in dem erfindungsgemäßen Verfahren die Verbindung II in einer Zusammensetzung erhalten, die die unmittelbare weitere Umsetzung, z.B. in einer Hydrierung, der Verbindung II und ggf. anderen Bestandteilen der Zusammensetzung ermöglicht, ohne dass ein oder mehrere weitere Aufreinigungsschritt(e) erforderlich sind. Dies ist insbesondere bei Berücksichtigung des Ausgangsmaterials "Fermentationsbrühe" überraschend. Vorteilhaft werden in dem erfindungsgemäßen Verfahren auch Derivate , wie z. B. Monoamid der Bersteinsäure, die in dem erhaltenen Gemisch enthaltend sind, zu Pyrrolidonen umgesetzt, so dass es überraschend zu einer einheitlichen Umsetzung des Succinimid kommt, trotz der vielen Nebenkomponenten, die die Fermentationsbrühe vor der Umsetzung laut dem erfindungsgemäßen Verfahren enthält.

Aufarbeitungen von Stoffwechselprodukten hergestellt in Fermentationen, bedürfen oftmals besonderer Reinigungsschritte, da nicht oder unvollständig umgesetzte Bestandteile des Mediums, Abbauprodukte von Bestandteilen des Mediums, gebildete Nebenprodukte und deren Abbauprodukte, durch lysierte Zellen freigesetzte Bestandteile und deren Abbauprodukte, wie z.B. Salze, Polysaccharide, Proteine, Peptide und Aminosäuren, Amine, Amide, organische Säuren, Alkohole, Aldehyde, und/oder Ketone, die jeweiligen Abbauprodukte und/oder andere organische und anorganische Verbindungen. eine weitere chemische Umsetzung inhibieren können oder nicht zu einem Reinigungseffekt führen, der eine Folgereaktion ohne Zwischenreinigung erlaubt. Vorteilhaft ist Verbindung I thermisch stabil. Im vorliegenden erfindungsgemäßen Verfahren entfallen zeitaufwändige und teuere Reinigungsschritte der Verbindung I, wie z.B. Chromatographie, Elektrodialyse oder Kristallisation. Vorteilhaft ist zudem, dass der gegebenenfalls entstehende Ammoniak in Form von wässriger Ammoniumhydroxid-Lösung zurückgewonnen und wieder in der Fermentation eingesetzt werden kann. Durch das erfindungsgemäße Verfahren kann die Gefahr der Katalysatorvergiftung durch Nebenbestandteile der Fermentationsbrühe herabgesetzt werden und die destillative Reinigung der Endprodukte erleichtert werden, weil die meisten Nebenprodukte aus der Fermentation bei der Destillation der Verbindung II oder der Zusammensetzung enthaltend die Verbindung II abgetrennt werden.

Folglich wird in einer bevorzugten Ausführungsform die Verbindung II und/oder die oben genannten Derivate ohne weitere Isolierung oder Zwischenreinigung umgesetzt, z.B. wie unten beschrieben, insbesondere in einer Hydrierung.

Folglich wird in einer weiteren Ausführungsform in dem vorliegenden Verfahren der entstehende Ammoniak zurückgewonnen und vorzugsweise wieder in der Fermentation eingesetzt, beispielsweise als wässrige Ammoniumhydroxid-Lösung.

Bei der Umsetzung von Verbindung I zu Verbindung II entstehen die Zwischenprodukte Verbindung IIa und Verbindung IIb: 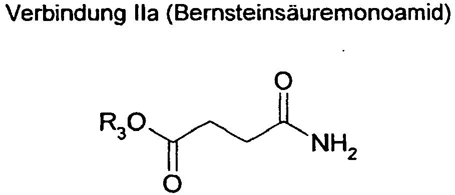 wobei R₃ gleich H⁺ oder ein anderes Kation sein kann, vorzugsweise ein Kation, das ein Mediumbestandteil ist, beispielsweise ein Kation wie oben aufgezählt oder NH₄⁺;
und/oder

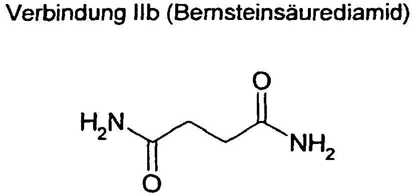

Je nach Wahl der Parameter kann in dem erfindungsgemäßen Verfahren der jeweilige Anteil von Verbindung II und/oder IIa und/oder IIb verändert und bestimmt werden In einer Ausführungsform sind die Verweilzeit im oberen Bereich des Temperaturintervalls länger und wird das entstehende Wasser und ggf. der entstehende Ammoniak im wesentlichen vollständig abdestilliert, beispielsweise zu mindestens 50%, vorzugsweise zu 60%, 70%, 80%, 90%, 95, 97%, 99% oder mehr. Wasser wird vorzugsweise vollständig abdestilliert. Folglich betrifft die Erfindung in einer Ausführungsform ein erfindungsgemäßes Verfahren in dem eine Zusammensetzung enthaltend Succinimid und/oder Bernsteinsäuremonoamid und/oder Bemsteinsäurediamid herstellt wird.

In einer Ausführungsform werden in dem erfindungsgemäßen Verfahren die Parameter so gewählt, dass eine Verbindung II enthaltende Schmelze erhalten wird. Folglich liegt in einer Ausführungsform die Temperatur über dem Schmelzpunkt der Schmelze. Der Schmelzpunkt von Succinimid liegt zwischen ungefähr 120°C bis 130°C, der Schmelzpunkt einer Succinimid-haltigen Schmelze liegt je nach Verunreinigungsgrad unterhalb des Schmelzpunkts von Succinimid. In einer Ausführungsform wird die Schmelze bei einer Temperatur größer als 120°C, beispielsweise größer als 126 °C gehalten.

In einer Ausführungsform findet das Destillieren von Wasser und/oder Ammoniak und ggf. sonstigen leichterflüchtigen Stoffen im wesentlichen parallel zur Umsetzung statt.

Erfindungsgemäß erfolgt in einer Ausführungsform die Destillation des Succinimids nach dem Destillieren von Wasser und Ammoniak.

In einer Ausführungsform betrifft die Erfindung eine Zusammensetzung, die Succinimid und weiterhin mindestens Bernsteinsäuremonoamid oder mindestens Bernsteinsäuremonoamid und Bernsteinsäurediamid enthält. Je nach Wahl der Verfahrensparameter entsteht in dem erfindungsgemäßen Verfahren ein Gemisch, das zu unterschiedlichen Anteilen Succinimid, Bernsteinsäure, Bernsteinsäuremonoamid und/oder Bemsteinsäurediamid enthält. Die restlichen Fermentationsnebenbestandteile sind vorzugsweise im wesentlichen abgetrennt. Vorteilhaft wird in dem erfindungsgemäßen Verfahren unabhängig davon in welchen Anteilen Succinimid sowie Bernsteinsäuremonamid und/oder Bemsteinsäurediamid nach Schritt (c) in dem Destillationssumpf enthalten sind, ein Produkt erhalten, das überraschenderweise Succinimid, Bernsteinsäuremonamid beziehungsweise Bemsteinsäurediamid in einer Form zur Verfügung stellen kann, die eine Umsetzung ohne weitere Aufarbeitung erlaubt.

In einer Ausführungsform erfolgt folglich die Umsetzung des Destillats aus Schritt (d) oder die Weiterverarbeitung des Destillats aus Schritt (d) ohne weitere Aufarbeitung. Insbesondere kann in einer Ausführungsform das Destillat aus Schritt (d) ohne weitere Aufarbeitung zu Pyrrolidonen, umgesetzt werden oder die zu Bernsteinsäure oder deren Salzen oder Bernsteinsäurederivaten rückgespalten werden.

Überraschenderweise kann das Produkt der Destillation aus Schritt (d), enthaltend Verbindung II, IIa und/oder IIb unmittelbar durch Reduktion zu Verbindung IIIa weiter umgesetzt werden.

Insbesondere wird überraschenderweise bei einer erfindungsgemäßen Reduktion einer Zusammensetzung enthaltend Verbindung II sowie IIa, und/oder IIb nicht nur Verbindung II sondern auch Verbindungen IIa und IIb, möglicherweise über die Zwischenstufe Verbindung II, zur Verbindung IIIa umgesetzt..

Folglich wird in einer Ausführungsform durch die hierin beschriebenen erfindungsgemäßen weiteren Verfahrensschritte das Destillat aus Schritt (d) mit hohen Ausbeuten zu 2-Pyrrolidon umgesetzt.

Optional kann die Verbindung II, beispielsweise Succinimid oder eine Zusammensetzung enthaltend Succinimid, Bernsteinsäure, Bernsteinsäuremonoamid und/oder -diamid aus der Schmelze weiter aufgearbeitet, z.B. weiter aufgereinigt, werden.

In einer Ausführungsform betrifft die vorliegende Erfindung folglich ein Verfahren zur Herstellung der Verbindung IIIa oder einer Zusammensetzung enthaltend die Verbindung IIIa 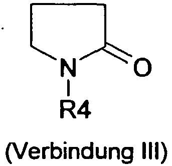 wobei R4 gleich H ist (Verbindung IIIa), oder
und wobei das Verfahren die Schritte des Verfahrens nach Anspruch 1 oder 2 enthält und weiterhin folgend:
(f) Reduzieren der Verbindung II zur Verbindung III.

Überraschenderweise kann also mit dem vorliegenden Verfahren eine Verbindung I, die in einer Fermentationsbrühe vorliegt, durch Umsetzung zur Verbindung II und einfache Destillation dergleichen bzw. der Verbindungen IIa und/oder IIb, unter Vermeidung einer kostenintensiven Aufreinigung von Verbindung I mittels Elektrodialyse, Kristallisation, Extraktion, etc., zur Verbindung IIIa bzw. IIIb umgesetzt werden.

Insbesondere können so vorteilhaft mit dem erfindungsgemäßen Verfahren wahlweise substituierte oder unsubstituierte Pyrrolidone hergestellt werden, insbesondere 2-Pyrrolidon. Folglich ist in einer Ausführungsform die Reduzierung wie unten beschrieben eine Hydrierung.

Besonders vorteilhaft ist, dass durch das erfindungsgemäße Verfahren kostengünstig und ohne zeitaufwendige Reinigungsschritte eine im wesentlichen 2-Pyrrolidon enthaltende Zusammensetzung bereitgestellt wird. In einer Ausführungsform enthält das erfindungsgemäß hergestellte 2-Pyrrolidon kein NMP.

In einer Ausführungsform betrifft die vorliegende Erfindung folglich ein Verfahren zur Herstellung einer Verbindung IIIb oder einer Zusammensetzung enthaltend die Verbindung IIIb, insbesondere einer Zusammensetzung enthaltend die Verbindung IIIa und IIIb 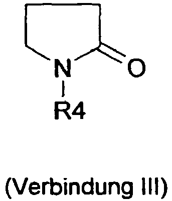 wobei für Verbindung IIIa R₄ gleich H ist; und
wobei für Verbindung IIIb R₄ gleich verzweigtes oder unverzweigtes Alkyl mit 1 bis 20 Kohlenstoffresten mit 0 bis 1 OH- oder NH₂-Resten ist;
und wobei das Verfahren die Schritte des Verfahrens nach Anspruch 3 enthält und weiterhin folgend:
(g) vollständiges oder partielles Alkylieren der Verbindung IIIa zur Verbindung IIIb während oder nach Schritt (f).

Um substituierte Pyrrolidone herzustellen, können geeignete Alkylierungsmittel, beispielsweise die weiter unten genannten Alkylierungsmittel, einer Hydrierung zugesetzt werden. Besonders bevorzugt wird zur Herstellung eines N-Alkyl-Pyrrolidons ein gerad- oder verzweigtkettiger C1 bis C20-Alkohol oder -Alkylamin eingesetzt, vorzugsweise ein gerad- oder verzweigtkettiger C1 bis C4-Alkohol, z.B. Methanol, Ethanol, Propanol oder Butanol, oder ein entsprechendes Amin, z.B. Methylamin, Ethylamin, Butylamin oder Propylamin. In einer Ausführungsform wird folglich in Gegenwart von Methanol oder Ethanol alkyliert.

In einer Ausführungsform wird folglich das in dem erfindungsgemäßen Verfahren hergestellte 2-Pyrrolidon zu substituierten Pyrrolidonen, beispielsweise Hydroxethylypyrrolidon oder N-Methylpyrrolidon (NMP), oder Gemischen davon, umgesetzt. Vorteilhaft kann durch die Wahl der Verfahrensparameter das 2-Pyrrolidon zu einem definierten Gemisch von 2-Pyrrolidon und substituierten Pyrrolidonen, insbesondere NMP, oder nahezu vollständig zu substituierten Pyrrolidonen umgesetzt werden. Durch die Wahl der Parameter kann der Grad der Umsetzung und somit die Verteilung der Produkte in dem Produktgemisch bestimmt werden. In einer Ausführungsform wird Verbindung II in Gegenwart des Alkylierungsmittels, z.B. eines Amins wie u.a. Alkylamin oder Hydroxyalkylamin oder Alkyldiamin, umgesetzt. Je höher das molare Verhältnis des Alkylierungsmittels, beispielsweise von Alkylamin oder Hydroxyalkylamin oder Alkyldiamin zu Verbindung II ist, desto mehr Verbindung IIIb entsteht im Gemisch mit IIIa.

In einer Ausführungsform des erfindungsgemäßen Verfahrens wird die Fermentationsbrühe hergestellt durch anaerobe oder aerobe Fermentation oder einer Kombination aus aerober und anaerober Fermentation, bevorzugt durch die Fermentation von Kohlenstoffquellen, wie beispielsweise Ölen oder Alkoholen, insbesondere Glycerin, Ethanol, Methanol, L-Sorbose oder D-Sorbit, oder Zuckern, wie z.B. C6- oder C5-Zuckem, insbesondere Glucose, Saccharose, Arabinose, Xylose oder CO₂, oder H₂, in Reinform oder z.B. als Melasse oder Mischungen der genannten Stoffe oder deren Vorprodukten, wie z. B. Stärke, beispielsweise zusammen mit Enzymen, oder anderen möglichen kohlenstoffhaltigen Zusammensetzungen wie z. B. Cellulose, z.B. in Form von Altpapier, Holzabfällen oder Bestandteilen von stärkehaltigen Pflanzen.

Zur Fermentation werden beispielsweise prokaryotische oder eukaryotische Mikroorganismen verwendet, beispielsweise Bakterien, wie E. coli, Anaerobiopirillum succiniproducens, Actinobacillus succinogenes, Mannheimia succiniproducens oder andere Bakterien oder Pilze, die Bernsteinsäure produzieren.

In einer Ausführungsform wird in der Fermentation ein Mikroorganismus, beispielsweise E.coli, nach einer aeroben Wachstumsphase zur Entwicklung von Biomasse zu anaeroben Bedingungen transferiert. In dieser anaeroben Phase findet die Synthese von Succinat statt. Beide Kultivierungsschritte können insbesondere in Komplexmedium stattfinden. In einer weiteren Ausführungsform erfolgt in der Fermentation die Kultivierung des Mikroorganismus, insbesondere von E. coli, in der anaeroben Phase mit Minimalmedium. Eine weitere Ausführungsform umfasst das mehrmalige Recycling der Zellen und Durchführung der anaeroben Produktionsphase auf Komplex- oder Minimalmedium.
Die Fermentation kann sowohl in Rührfermentem, Blasensäulen und Schlaufenreaktoren durchgeführt werden. Ein ausführlicher Überblick über die möglichen Ausführungsarten inkl. Rührerformen und geometrischer Ausführungen ist in "Chmiel: Bioprozesstechnik: Einführung in die Bioverfahrenstechnik, Band 1" zu finden. Bei der Prozessführung stehen typischerweise die folgenden, dem Fachmann bekannten oder z. B. in "Chmiel, Hammes und Bailey: Biochemical Engineering" erläuterten Varianten wie Batch, Fed-Batch, Repeated Fed-Batch oder aber auch kontinuierliche Fermentation mit und ohne Rückführung der Biomasse zur Verfügung. Je nach Produktionsstamm kann/muss eine Begasung mit Sauerstoff, Kohlendioxid, Wasserstoff oder Stickstoff erfolgen, um gute Ausbeuten zu erzielen.

Vor der Umsetzung in der Fermentationsbrühe in dem erfindungsgemäßen Verfahren kann die Fermentationsbrühe vorbehandelt werden, beispielsweise kann die Biomasse der Brühe abgetrennt werden. Verfahren zur Abtrennung der Biomasse sind dem Fachmann bekannt, z.B. Filtration, Sedimentation und Flotation. Folglich kann die Biomasse beispielsweise mit Zentrifugen, Separatoren, Dekantern, Filtern oder in Flotationsapparaturen abgetrennt werden. Zur möglichst vollständigen Gewinnung des Wertproduktes empfiehlt sich oftmals ein Waschen der Biomasse, z. B. in Form einer Diafiltration. Die Wahl der Methode ist abhängig von dem Biomassenanteil in der Fermenterbrühe und den Eigenschaften der Biomasse, sowie der Interaktion der Biomasse mit dem Wertprodukt.

Die Fermentationsbrühe kann in einer Ausführungsform sterilisiert oder pasteurisiert werden.

In einer weiteren Ausführungsform wird die Fermentationsbrühe aufkonzentriert. Diese Aufkonzentrierung bzw. Eindampfung kann je nach Anforderung diskontinuierlich, oder kontinuierlich geschehen. Dabei sollten Druck- und Temperaturbereich so gewählt werden, dass zum einen keine Produktschädigung auftritt, zum anderen ein minimierter Einsatz von Apparaten und Energie notwendig ist. Insbesondere die geschickte Wahl der Druck- und Temperaturstufen für eine mehrstufige Verdampfung ermöglicht eine Einsparung von Energie.
Apparativ können dazu Rührkessel, Fallfilm-, Dünnschicht, Zwangsentspannungsumlauf-, und sonstige Verdampferbauarten in Natur- oder Zwangsumlauffahrweise genutzt werden.

Folglich wird unter dem Begriff "Fermentationsbrühe" eine auf einen fermentativen Prozess beruhende wässrige Lösung verstanden, die nicht oder beispielsweise wie hierin beschrieben aufgearbeitet wurde.

Je nach Wahl der Verfahrensparameter entsteht in dem erfindungsgemäßen Verfahren bei der Umsetzung und Destillation nach Schritt (b) ein Gemisch das u.a. die Produkte Bernsteinsäure, Bernsteinsäuremonoamid, Succinimid und Bernsteinsäurediamid enthält. Der Anteil der verschiedenen Produkte kann z.B. durch die Wahl der Verweilzeit, Temperatur, oder des Wasser- und Ammoniakanteils verändert werden. Die restlichen Fermentationsnebenbestandteile werden im wesentlichen bei der Destillation abgetrennt. Überraschenderweise kann das Gemisch aus Bernsteinsäure, Bemsteinsäuremonoamid, Succinimid und Bemsteinsäurediamid mit hohen Ausbeuten zu Pyrrolidonen umgesetzt werden, insbesondere zu 2-Pyrrolidon. In einer Ausführungsform des erfindungsgemäßen Verfahrens werden die Reaktionsparameter der Umsetzung so gewählt werden, dass eine im wesentlichen Succinimid enthaltende Schmelze entsteht wenn die Temperatur des Destillationsaustrages über dem Schmelzpunkt, vorzugsweise auf > 130°C gehalten wird.

Das Verfahren zur Herstellung von Succinimid kann kontinuierlich oder im batch durchgeführt werden.

In einem kontinuierlichen Verfahren wird das Umsetzen und Destillieren bei folgenden Bedingungen durchgeführt:
(i) vorzugsweise Abtrennen der Biomasse und/oder Aufreinigen der Fermentationsbrühe;
(ii) vorzugsweise Aufkonzentieren der Fermentationsbrühe;
(iii) Abdestillieren der Nebenprodukte, z.B. H₂O und NH₃, und Umsetzung der Verbindung I in der Fermentationsbrühe bei einer Temperatur von beispielsweise ungefähr 100 bis ungefähr 300°C, mehr bevorzugt bei ungefähr 150 bis ungefähr 200°C, am meisten bevorzugt bei ungefähr 170°C, beispielsweise bei Normaldruck, bis eine Schmelze vorliegt; und
(iv) vorzugsweise, Nachreagieren bei beispielsweise ungefähr 150 bis ungefähr 300°C, bevorzugt ungefähr 200 bis ungefähr 300°C, besonders bevorzugt bei ungefähr 250°C, beispielsweise für im wesentlichen weniger als ungefähr 2 h, beispielsweise für ungefähr 0,5 h bis ungefähr 1 h und beispielsweise bei Normaldruck; und
(v) Abdestillieren von Verbindung II sowie ggf. Verbindung IIa und IIb, beispielsweise bei einer Temperatur von ungefähr 150°C bis 300°C, vorzugsweise bei mehr als 170°C bzw. weniger als 270°C, mehr bevorzugt bei ungefähr 220°C bis ungefähr 250°C, wobei die Temperatur natürlich neben dem Druck von der Zusammensetzung, der Nebenkomponenten und dem Abtrenngrad stark abhängt, und wobei beispielsweise bei Normaldruck oder Unterdruck, bevorzugt bei einem Druck von 0,01- bis 1000 mbar, bevorzugt 1 - 100 mbar, beispielsweise zum Abreinigen von Nebenkomponenten, erfolgt.

In einer Ausführungsform wird das Umsetzen und Destillieren batch-weise bei folgenden Bedingungen durchgeführt:
(i) vorzugsweise Abtrennen der Biomasse und/oder Aufreinigen der Fermentationsbrühe;
(ii) vorzugsweise Aufkonzentieren der Fermentationsbrühe;
(iii) Abdestillieren der Nebenprodukte, z.B. H₂O und NH₃, und Umsetzen der Verbindung I bei einer Temperatur von RT bis ungefähr 300 °C, bevorzugt bei Normaldruck; und
(iv) vorzugsweise, Nachreagieren bei beispielsweise ungefähr 150 bis ungefähr 300°C, bevorzugt ungefähr 200 bis ungefähr 300°C, besonders bevorzugt bei ungefähr 250°C für beispielsweise weniger als ungefähr 2 h, mehr bevorzugt für ungefähr 0,5 h bis ungefähr 1 h und beispielsweise bei Normaldruck; und
(v) vorzugsweise, folgendes Überdestillieren des Reaktionsgemisches bei verringertern Druck und bei ca. 150 bis ca. 300 °C bevorzugt 170 - 250 °C, beispielsweise zum Abreinigen von Nebenkomponenten durch Destillation, besonders vorzugsweise bei einer Temperatur von beispielsweise ungefähr 185 bis ungefähr 195°C und beispielsweise bei Normaldruck oder bei Unterdruck, bevorzugt bei einem Druck von ungefähr 0,01 bis ungefähr 1000 mbar bevorzugt bei 1 - 100 mbar, besonders bevorzugt bei ungefähr 25 mbar.

In einer Ausführungsform enthält das erfindungsgemäße Verfahren alle genannten Verfahrensschritte (i) bis (iii), insbesondere bei dem genannten Druck und bei der genannten Temperatur. Bevorzugt wird das Verfahren unter den jeweils bevorzugten Bedingungen durchgeführt.

Die Reaktion nach Schritt (i) kann beispielsweise in Rührkesseln oder sonstigen Reaktoren, in verschiedenen Verdampferarten wie z. B. Fallfilmverdampfern, in einer Destillationskolonne oder in Kombinationen davon erfolgen.
Auch ein Sprühtrockner oder ein Sprühgranulator kann als Reaktiv-Trockner genutzt werden, wobei das Produkt als Feststoff vorliegt, der danach zur Aufreinigung bzw. Weiterreaktion wieder aufgeschmolzen bzw. gelöst werden muss.

Als Nebenkomponenten werden bevorzugt Acetat, Lactat, Formiat und Medienbestanteile (z. B. Salze, Proteine, Zucker, Aminosäuren, Hefeextrakt in Schritt (iii) abgetrennt. Vorzugsweise beträgt der Anteil an Succinimid je nach Ausgangsmaterial mehr als 50 %, 60 % oder 70 %, vorzugsweise mehr als 80 %, noch mehr bevorzugt mehr als 85 %. So wird beispielsweise bei kontinuierlicher Fahrweise mit Pluriol P600 und Minimalmedium versetztem DAS eine Reinheit von 85 % oder mehr Succinimid erreicht, und bei Einsatz von Fermentationsbrühe nach Biomassenabtrennung (mit Minimalmedium fermentiert) und mit synth. DAS aufgestockt 87 % oder mehr Succinimid.

Bei batch-Fahrweise kann beim Einsatz von mit Minimalmedium aufgestocktem DAS eine Reinheit von 70 - 98 % Succinimid oder mehr erreicht werden.

Alternativ zur Umsetzung zu Succinimid und dessen Destillation kann das Abreinigen des Diammoniumsuccinats an Nebenkomponenten auch über eine Freisetzung der Bernsteinsäure und Kristallisation, über Membranverfahren insbesondere Elektrodialyse, über Chromatographie oder Extraktionoder anderen dem Fachmann bekannten Verfahren erreicht werden.

In einer Ausführungsform wird der Fermentationsbrühe ein 1-50%, vorzugsweise weniger als 30% Polyalkylenglykole, wie z. B. Pluriol (Handelsname), insbesondere Pluriol P600 (Handelsname), oder Silikonöle, wie z. B. Baysilone (Handelsname) oder Paraffinöle zugegeben
Folglich betrifft in einer Ausführungsform die vorliegende Erfindung eine Zusammensetzung, beispielsweise eine Schmelze, enthaltend Verbindung II und Polyalkylenglykole, wie z. B. Pluriol (Handelsname), insbesondere Pluriol P600 (Handelsname), oder Silikonöle, wie z. B. Baysilone (Handelsname) oder Paraffinöle, beispielsweise mit Pluriol P600.
Vorzugsweise wird soviel der vorstehend bezeichneten Verbindungen verwendet, dass die Reaktion und Destillation durch ausreichende Erniedrigung der Viskosität und Erhaltung der Rührbarkeit des Reaktionsgemisches verbessert wird.

Gerade bei der Herstellung von substituierten oder nicht-substituierten Pyrrolidonen ist die Herstellung von Succinimid aus DAS gemäß dem erfindungsgemäßen Verfahren vorteilhaft, da auch bei einem nicht vollständigen Umsatz die Mischung aus DAS, Bernsteinsäuremonoamid und -diamid sowie Succinimid weiterverarbeitet werden kann. Folglich erfolgt in einer Ausführungsform des erfindungsgemäßen Verfahrens die Reduzierung der Verbindung II oder der Zusammensetzung enthaltend die Verbindung II und optional die Verbindungen IIa und IIb zur Verbindung III durch eine Hydrierung, beispielsweise eine katalytische Hydrierung mit Wasserstoff in Gegenwart von homogenen oder heterogenen Katalysatoren, die z.B. suspendiert oder fest angeordnet sein können, oder eine Hydrierung mit komplexen Hydriden, oder eine Transferhydrierung bei der z.B. Alkohole wie Methanol oder iso-Propanol unter Bildung von Aldehyd oder mit einem Keton, das Wasserstoff zum Hydrieren abgibt.

In einer bevorzugten Ausführungsform ist die Reduzierung der Verbindung II eine katalytische Hydrierung und kann in der Gas- oder der Flüssigphase durchgeführt werden.

Als Reaktoren kommen alle für Hydrierungen übliche Typen in Frage, wie zum Beispiel batch betriebene, z.B. gerührte, oder kontinuierlich betriebene Reaktoren: Als Reaktoren können z.B. Rohrreaktoren, Rohrbündelreaktoren, Schachtreaktoren oder Wirbelbettreaktoren verwendet werden.

Die Hydrierung kann in einer Stufe erfolgen oder mehrstufig, z.B. mit Haupt- und Nachreaktor. Dabei wird bevorzugt der Hauptreaktor, zumindest in der Flüssigphase, mit äußerem Umlauf zur Wärmeabfuhr betrieben, der Nachreaktor bevorzugt im geraden Durchgang, um möglichst hohen Umsatz zu erreichen. Als Reaktionsdruck in der Flüssig- oder Gasphase können die unten erwähnten Drücke.eingesetzt werden.

Bei der Herstellung von Pyrrolidon kann die Hydrierung unter Zusatz von Ammoniak erfolgen, es ist aber nicht notwendig. Wird Ammoniak zugesetzt, so kann die Bildung von gamma-Butyrolacton zurückgedrängt werden, was die Aufarbeitung der Reaktionsausträge erleichtert. Bezogen auf eingesetztes Edukt wird Ammoniak bis zu ungefähr 10fachen, bevorzugt bis 5fachen, besonders bevorzugt bis 2fachen molaren Überschuss eingesetzt.

Bei der Aufarbeitung können anfallende Produkte, die noch nicht vollständig umgesetzt sind, generell rückgeführt werden, um die Ausbeute zu erhöhen. Solche Produkte sind z.B. Imide oder Bisamide.

Als Hydrierkatalysatoren können im erfindungsgemäßen Verfahren im Allgemeinen heterogene, aber auch homogene, zur Hydrierung von Carbonylgruppen geeignete Katalysatoren Verwendung finden. Sie können sowohl fest angeordnet, als auch mobil, wie z.B. in einem Wirbelbettreaktor, eingesetzt werden. Beispiele hierfür sind z.B. in Houben-Weyl, Methoden der Organischen Chemie, Band IV/1c, S. 16 bis 26, beschrieben. Von diesen Hydrierkatalysatoren sind solche bevorzugt, die ein oder mehrere Elemente der Gruppe 11, 6, 7, 8, 9, 10, 13, 14 und 15 des Periodensystems der Elemente, insbesondere Kupfer, Rhenium, Mangan, Kobalt, Ruthenium, Rhodium, Nickel, Palladium, Eisen, Platin, Indium, Zinn und Antimon enthalten. Besonders bevorzugt sind Katalysatoren, die Kupfer, Kobalt, Ruthenium oder Rhenium enthalten.

Die im erfindungsgemäßen Verfahren eingesetzten Katalysatoren können z.B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z.B. schwerlösliche Hydroxide, Oxydhydrate, basische Salze oder Carbonate ausfällt, die erhaltenen Niederschläge anschließend trocknet und diese dann durch Calcinierung bei im allgemeinen ungefähr 300 bis ungefähr 700°C, insbesondere ungefähr 400 bis ungefähr 600°C in die entsprechenden Oxide, Mischoxide und/oder gemischtvalentigen Oxide umwandelt, welche durch eine Behandlung mit Wasserstoff oder mit Wasserstoff enthaltenden Gasen bei in der Regel ungefähr 50 bis ungefähr 700°C, insbesondere ungefähr 100 bis ungefähr 400°C zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten z.B. durch Imprägnierung auf ein Trägermaterial aufgebracht worden sind.
Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, dass höhere Gehalte diese Trägerkatalysatoren an katalytisch aktiven Metallen zu höheren Raum-Zeit-Umsätzen führen können als niedrigere Gehalte. Im Allgemeinen werden Trägerkatalysatoren verwendet, deren Gehalt an katalytisch aktiven Metallen ungefähr 0,1 bis ungefähr 90 Gew.-%, vorzugsweise ungefähr 0,5 bis ungefähr 40 Gew.-% bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert. Als Trägermaterialien können im allgemeinen die Oxide des Aluminiums und Titans, Zirkoniumdioxid, Siliciumdioxid, Tonerden, z.B. Montmorillonite, Silikate wie Magnesium- oder Aluminiumsilikate, Zeolithe wie ZSM-5 oder ZSM-10-Zeolithe, sowie Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren anwendbaren Katalysatoren dienen.

Als für im erfindungsgemäßen Verfahren einsetzbaren Heterogenkatalysatoren seien die folgenden beispielhaft genannt: Kobalt auf Aktivkohle, Kobalt auf Siliciumdioxid, Kobalt auf Aluminiumoxid, Rhenium auf Aktivkohle, Rhenium auf Siliciumdioxid, Rhenium/Zinn auf Aktivkohle, Rhenium/Platin auf Aktivkohle, Kupfer auf Aktivkohle, Kupfer/Siliciumdioxid, Kupfer/Aluminiumoxid, Kupferchromit, Bariumkupferchromit, Kupfer/Aluminiumoxid/Manganoxid, Kupfer/Aluminiumoxid/Zinkoxid sowie die Katalysatoren gemäß DE-A 3 932 332, US-A 3 449 445, EP-A 44 444, EP-A 147 219, DE-A 3 904 083, DE-A 2 321 101, EP-A 415 202, DE-A 2 366 264, EP 0 552 463 und EP-A 100406.

Zur Herstellung von substituierten Pyrrolidonen ausgehend von Succinimid und Diammoniumsuccinat kann die Hydrierung unter Zusatz von den entsprechenden Alkoholen oder Aminen erfolgen.

In einer weiteren Ausführungsform wird in dem erfindungsgemäßen Verfahren 2-Pyrrolidon in einer Gasphase oder in einer flüssigen Phase alkyliert. In einer bevorzugten Ausführungsform wird 2-Pyrrolidon in der Gasphase zu einem verzweigten oder unverzweigten C1 bis C20-N-Alkylpyrrolidon oder verzweigten oder unverzweigten N-Hydroxyalkylpyrrolidon oder verzweigtem oder unverzweigtem Aminoalkylpyrrolidonalkyliert, besonders bevorzugt ist die Alkylierung zu N-Methylpyrrolidon (NMP) und N-Ethylpyrrolidon. Dabei wird Pyrrolidon bei Temperaturen von 100 bis 400 °C, bevorzugt 140 bis 370 °C, besonders bevorzugt bei 180 bis 350 °C und Drücken (absolut) zwischen 0,2 und 50 bar bevorzugt 0,7 bis 40 bar, besonders bevorzugt 0,8 bis 20 bar mit Alkoholen, Alkylaminen, Hydroxyalkylaminen oder Dialkylaminen umgesetzt. Alkohole sind bevorzugt. Das Alkylierungsmittel wird dabei bevorzugt im Überschuss zugegeben, d.h. bezogen auf Pyrrolidon 1,01 bis 10 Moläquivalente. Bevorzugt sind 1,1 bis 3 Moläquivalente. Dabei kommen Katalysatoren zum Einsatz, die saure oder basische Zentren aufweisen. Üblicherweise sind dies oxidische Katalysatoren wie z.B. Aluminiumoxid, Siliziumoxid, Titanoxid oder auch gemischte Oxide wie z.B. Tonerden, Zeolithe oder dergleichen.

Um substituierte Pyrrolidone herzustellen, können insbesondere auch die weiter unten genannten Alkylierungsmittel der Hydrierung zugesetzt werden. Besonders bevorzugt wird zur Herstellung eines N-Alkyl-Pyrrolidons ein gerad- oder verzweigtkettiger C1 bis C20-Alkohol oder -Alkylamin eingesetzt, vorzugsweise ein gerad- oder verzweigtkettiger C1 bis C4-Alkohol, z.B. Methanol, Ethanol, Propanol oder Butanol, oder ein entsprechendes Amin, z.B. Methylamin, Ethylamin, Butylamin oder Propylamin. In einer Ausführungsform wird folglich in Gegenwart von Methanol oder Ethanol alkyliert.

Will man beispielsweise NMP herstellen, so kann in Gegenwart von Methanol oder Methylamin hydriert werden. Je höher der Überschuss an Alkylierungsmittel ist, desto höher wird der Anteil an dem entsprechenden Produkt. Sofern es nicht erwünscht ist, Gemische aus Pyrrolidon und dem entsprechenden substituierten Pyrrolidon zu erhalten, kann Pyrrolidon nach der destillativen Aufarbeitung in die Reaktion zurückgeführt werden.

In einer Ausführungsform wird in dem erfindungsgemäßen Verfahren Succinimid zu 2-Pyrrolidon hydriert.

In einer Ausführungsform wird die Reduzierung eine Gas- oder Flüssigphasenhydrierung, die bei folgenden Bedingungen durchgeführt wird:
(i) bei einer Temperatur zwischen ungefähr 80°C und ungefähr 330°C, bevorzugt zwischen ungefähr 120°C und ungefähr 300°C, besonders bevorzugt zwischen ungefähr 150°C und ungefähr 280°C;
(ii) mit einem homogenen oder heterogenen Katalysator geeignet zur Hydrierung von Carbonylgruppen, vorzugsweise eines Katalysators der ein oder mehrere Elemente der Gruppe 11, 6, 7, 8-10, 13, 14 oder 15 des Periodensystems der Elemente enthält, bevorzugt ist ein Katalysator auf Basis von Kupfer, Rhenium, Mangan, Kobalt, Ruthenium, Rhodium, Nickel, Palladium, Eisen, Platin, Indium, Zinn oder Antimon, besonders bevorzugt auf Basis von Kupfer, Kobalt, Ruthenium oder Rhenium;
(iii) bei einem Druck in der Gasphase zwischen ungefähr Normaldruck und ungefähr 130bar, bevorzugt zwischen ungefähr 2 und ungefähr 100bar und besonders bevorzugt zwischen ungefähr 5 und ungefähr 60 bar oder bei einem Druck in der Flüssigphase zwischen ungefähr 20 und ungefähr 400bar, bevorzugt zwischen ungefähr 40 und ungefähr 300bar, besonders bevorzugt zwischen ungefähr 120 und ungefähr 290bar, und/oder
(iv) in Abwesenheit oder in Gegenwart von Ammoniak, bevorzugt in Gegenwart eines bis zu ungefähr 10fachen Überschusses, mehr bevorzugt in Gegenwart eines bis zu ungefähr 5fachen Überschusses, besonders bevorzugt in Gegenwart eines bis zu ungefähr 2fachen Überschusses von Ammoniak bezogen auf das Edukt.

In einer Ausführungsform wird das Verfahren bei dem genannten Druck und bei der genannten Temperatur und mit dem genannten Katalysator durchgeführt. Bevorzugt wird das Verfahren unter den jeweils am meisten bevorzugten Bedingungen durchgeführt.

Folglich erfolgt in einer Ausführungsform die Hydrierung in der Gasphase bei ungefähr 150°C bis ungefähr 280 °C und bei ungefähr 5 bis ungefähr 60 bar unter Katalyse eines Katalysators auf Basis von Kupfer, Kobalt, Ruthenium oder Rhenium und in Anwesenheit von ungefähr 1 bis ungefähr 2fachen Überschusses an Ammoniak bezogen auf das Edukt.

In einer weiteren Ausführungsform der vorliegenden Erfindung enthält das erfindungsgemäße Verfahren folgenden Schritt: Alkylieren der Verbindung IIIa mit einem verzweigten oder unverzweigten gesättigten Kohlenwasserstoff mit 1 bis ungefähr 20 Kohlenstoffresten und 1 bis 2 OH- oder NH₂-Resten, bevorzugt sind 1 oder 2 Kohlenstoffreste und 1 OH- oder NH₂-Rest.

Die Alkylierung kann in einer flüssigen Phase oder in einer Gasphase erfolgen.

Beispielsweise kann das Alkylieren in der Gasphase bei folgenden Bedingungen durchgeführt wird:
(i) bei einer Temperatur von ungefähr 50 bis ungefähr 600°C, bevorzugt bei ungefähr 100 bis ungefähr 500°C, besonders bevorzugt sind ungefähr 150 bis ungefähr 450°C;
(ii) bei einem Druck von ungefähr Normaldruck bis ungefähr 100bar, bevorzugt sind ungefähr Normaldruck bis ungefähr 50bar, besonders bevorzugt sind ungefähr Normaldruck bis 40bar;
(iii) Einsetzen der Alkylierungskomponente stöchiometrisch oder überstöchiometrisch, bevorzugt überstöchiometrisch, besonders bevorzugt in einem Verhältnis von ungefähr 1 bis ungefähr 10 Mol/Mol Pyrrolidon, besonders bevorzugt ungefähr 1 bis ungefähr 5 Mol/Mol Pyrrolidon; und
(iv) Verwenden eines basischen, neutralen oder lewis- bzw. brönstedsauren Oxids oder Mischoxid oder gemischten Oxids oder eines Metalls, bevorzugt von Ruthenium, Kobalt, Nickel oder Kupfer, als Katalysator.

Beispielsweise kann erfindungsgemäße die Alkylierung in der flüssigen Phase bei folgenden Bedingungen durchgeführt wird:
(i) bei einer Temperatur von ungefähr 50 bis ungefähr 600°C, bevorzugt bei ungefähr 100 bis ungefähr 500°C, besonders bevorzugt sind ungefähr 150 bis ungefähr 450°C;
(ii) bei einem Druck von Normaldruck bis ungefähr 325bar, bevorzugt sind ungefähr 10 bis ungefähr 250bar, besonders bevorzugt sind ungefähr 20 bis ungefähr 200bar;
(iii) Einsetzen der Alkylierungskomponente stöchiometrisch oder überstöchiometrisch, bevorzugt überstöchiometrisch, besonders bevorzugt in einem Verhältnis von ungefähr 1 bis ungefähr 10 Mol/Mol Pyrrolidon, besonders bevorzugt ungefähr 1 bis ungefähr 5 Mol/Mol Pyrrolidon; und
(iv) Verwenden eines basischen, neutralen oder lewis- bzw. brönstedsauren Oxids oder Mischoxid oder gemischten Oxids oder eines Metalls, bevorzugt von Ruthenium, Kobalt, Nickel oder Kupfer, als Katalysator.

Besonders bevorzugt wird das Alkylieren in der Flüssigphase durchgeführt.

In einer Ausführungsform wird das Verfahren bei dem genannten Druck und bei der genannten Temperatur und mit dem genannten Katalysator durchgeführt. Bevorzugt wird das Verfahren unter den jeweils am meisten bevorzugten Bedingungen durchgeführt.

Sofern der Umsatz bei der Alkylierung nicht vollständig ist, können die Edukte, nach destillativer Reinigung (z.B. batch oder kontin. Destillation), rückgeführt werden. Dabei werden üblicherweise Leichtsieder, wenn vorhanden, wie Wasser, Amin, Ammoniak, Alkohol in einer ersten Kolonne zuerst abgetrennt und ggf. rückgeführt, ggf wiederum nach Aufreinigung. Der verbleibende produktführende Strom wird dann, ggf. in einer weiteren Kolonne, weiter aufgetrennt, wobei, wenn vorhanden, nicht umgesetzes Pyrrolidon der Formel III abgetrennt und zurückgeführt wird.

In einer Ausführungsform wird in dem erfindungsgemäßen Verfahren ein substituiertes oder unsubstituiertes Pyrrolidon hergestellt. In einer Ausführungsform werden die genannten Schritte kombiniert, um ein N-Hydroxalkylpyrrolidon oder ein N-Alkylpyrrolidon, beispielsweise N-Methylpyrrolidon (NMP), herzustellen. In einer Ausführungsform wird ein Gemisch aus 2-Pyrrolidon und N-Methylpyrrolidon (NMP) hergestellt.

Folglich betrifft die vorliegende Erfindung in einer Ausführungsform ein Verfahren, das die folgenden Schritte enthält:
Fermentative Herstellung einer DAS-Lösung mit E. coli, Anaerobiopirillum succiniproducens, oder Actinobacillus succinogenes als Produktionsstamm aus Glucose.Abtrennen der Biomasse, Sterilisieren oder Pasteurisieren und Einengen der Fermentationsbrühe.
Ringschluss des DAS in der Lösung zu Succinimid bei gleichzeitiger Verdampfung von H₂O und NH₃ und Reinigung von Succinimid durch Destillation unter nahezu vollständiger Abtrennung der.bei der Hydrierung störenden Nebenkomponenten, wobei die Umsetzung und Reinigung in einem kontinuierlichen Verfahren bei folgenden Bedingungen durchgeführt wird: Abdestillieren von H₂O und NH₃ bei ungefähr Normaldruck bis bei ca. 150°C eine Schmelze vorliegt, die dann gegebenenfalls zur Nachreaktion weiter bis auf ungefähr 250°C aufgeheizt wird. Abreinigen von Nebenkomponenten durch Destillation bei einer Temperatur von ungefähr 190°C und bei einem Druck von ungefähr 20 bis ungefähr 30mbar. Im nächsten Schritt wird das hergestellte Succinimid und Bernsteinsäuremonoamid und/oder -diamid durch katalytische Hydrierung in der Schmelze bei ungefähr 250°C und ungefähr 250 bar in der Flüssigphase zu 2-Pyrrolidon umgesetzt mit einem homogenen oder heterogenen Katalysator geeignet zur Hydrierung von Carbonylgruppen, vorzugsweise auf Basis von Kupfer, Kobalt, Ruthenium oder Rhenium; in Gegenwart eines bis zu ungefähr 2fachen Überschusses von Ammoniak bezogen auf das Edukt. Im nächsten Schritt kann das hergestellte 2-Pyrrolidon in der Gasphase bei einer Temperatur von ungefähr 150 bis ungefähr 450°C; bei einem Druck von Normaldruck bis ungefähr 6 bar, mit Methanol in einem Verhältnis von mehr als 1 bis ungefähr 5 Mol/Mol Pyrrolidon; unter Katalyse eines oxidischen Katalysators, z.B. enthaltend Ruthenium, Kobalt, Nickel, Kupfer oder kein Metallzusatz, zu N-Methylpyrrilidon alykliert werde.

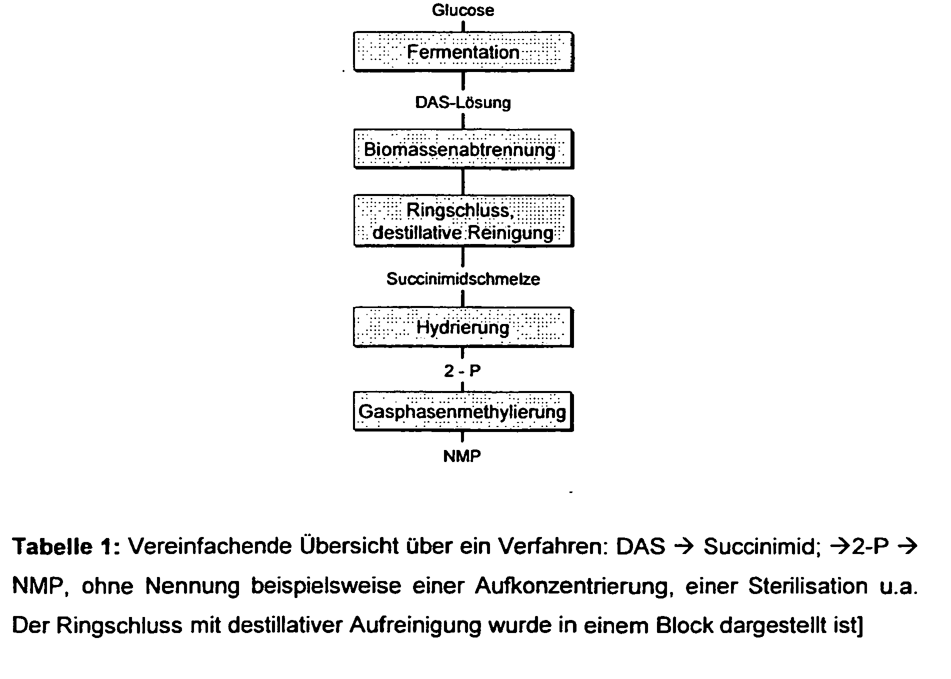

Die folgenden Beispiele erläutern die Erfindung und sollen nicht beschränkend interpretiert werden.

### Beispiele:

### Beispiel 1: Fermentative Herstellung von Bernsteinsäure [

Medien und Kultivierungsbedingungen: Vemuri, Eiteman und Altman, 2002, ergänzt wurde Ampicillin im Medium und das pH-Korrekturmittel NH4OH

### 1.1 Bereitung von "master"-Zellbank und Arbeitszellbank

Eine Probe des Succinatproduktionsstamms wird auf LB+Glucose-Agar ausgestrichen und für 20 Stunden bei 37 °C inkubiert und dann bei +4°C aufbewahrt. Ausgewählte Kolonien werden dann in LB+Glucose-Flüssigmedium weiter vermehrt. LB+Glucose-Flüssigmedium hat folgende Zusammensetzung: 10 g/l Tryptone, 5 g/l Hefeextrakt, 10 g/l NaCl und 10 g/l Glucose. LB+Glucose-Agar enthält zusätzlich 15 g/l Agar. Vorgefertigte Zubereitungen können von der Firma Becton Dickinson GmbH, Heidelberg als Bacto LB Broth, Miller oder Bacto LB-Agar, Miller bezogen werden. Nach Zusatz von 10 g/l Glucose erhält man die angegebenen Medien. Kulturen von 20 ml, die in 100 mL-Erlenmeyerkolben enthalten waren, werden für 15 Stunden bei 37°C und 180 rpm auf einem Schüttelinkubator (Firma Infors, Typ Multitron) inkubiert. Anschließend wird die Zellsuspension mit einer Laborzentrifuge (Firma Heraeus, Typ Biofuge Primo R; Rotor #7590, 50mL Greiner PP-Zentrifugenröhrchen, steril) für 15 Minuten bei 8500 rpm abzentrifugiert. Das Zellpellet wird in 10 mL sterilem LB+Glucose-Flüssigmedium, das mit 20 % Glycerin supplementiert ist, resuspendiert. Die resultierende Zellsuspension wird unter sterilen Bedingungen in Aliquots zu 1 ml abgefüllt und bei -70°C gelagert. Diese Kulturen werden als "master"-Zellbank (master cell bank) verwendet. Zur Herstellung einer Arbeitszellbank (working cell bank) wird steriles LB+Glucose-Flüssigmedium unter sterilen Bedingungen in 20 ml-Portionen in sterile 100 ml-Erlenmeyerkolben verteilt. Anschließend werden diese Kolben unter sterilen Bedingungen mit 100 µl der oben beschriebenen "master"-Zellbank Inokuliert. Die Inkubation erfolgt für 15 Stunden bei 37 °C und 180 rpm auf einem Schüttelinkubator (Firma Infors, Typ Multitron). Anschließend wird die Zellsuspension mit einer Laborzentrifuge (Firma Heraeus, Typ Biofuge Primo R; Rotor #7590, 50mL Greiner PP-Zentrifugenröhrchen, steril) für 15 Minuten bei 8500 rpm abzentrifugiert. Das Zellpellet wird in 10 mL sterilem LB+Glucose-Flüssigmedium, das mit 20 % Glycerin supplementiert ist, resuspendiert. Die resultierende Zellsuspension wird unter sterilen Bedingungen in Aliquots zu 1 ml abgefüllt und bei -70°C gelagert. Diese Kulturen werden als Arbeitszellbank (working cell bank) verwendet.

### 1.2 Herstellung einer Succinat-haltigen Fermentationsbrühe

Ein 1,0 ml-Aliquot der Arbeitszellbank (working cell bank) des Succinatproduktionsstamms wird dem Gefrierfach entnommen und 10 Minuten bei Raumtemperatur gelagert. 100 µl davon werden steril entnommen und auf einer LB+Glucose-Agarplatte mit einer Impföse steril verteilt. Nach Inkubation für 20 Stunden bei 37°C wird der Platte Zellmaterial steril entnommen und zur Inokulation der Vorkulturkolben (2000 ml-Rundkolben mit 4 Schikanen, die 300 ml Vorkulturmedium enthalten) verwendet. Die Vorkulturkolben werden für sechs Stunden bei 37 °C und 110 rpm auf einem Inkubationsschüttler (Firma Infors, Typ Multitron) inkubiert.
Für den Start der Hauptkultur werden 3,5 I Batchmedium mit 250 ml der Vorkultur beimpft. Die Kultivierung erfolgt für 120 h bei 37 °C in einem gerührten 5 I-Bioreaktor (Firma Infors, Typ ISF 100) mit einem 6-Blattscheibenrührer und vier Strombrechem, pH- und pO₂-Elektroden. Innerhalb der ersten 5,5 h (aeorbe Wachstumsphase) erfolgt die Kultivierung bei einer konstanten Drehzahl von 900 rpm und Begasung mit steriler Luft (3,0 l/min), danach wird auf 250 rpm und Begasung mit sterilem CO₂-Gas (0,18 l/min) umgestellt. Der pH-Wert wird zu Beginn mit 20 % NaOH-Lösung und 20 % HCl-Lösung auf 7,0 geregelt. Ab 5,5 h wird statt NaOH-Lösung 25 % NH4OH-Lösung verwendet und auf pH 6,8 geregelt: Die Dosierung des Feed-Mediums erfolgt nach einem festgelegten Dosierprofil, das gewährleistet, dass die Glucosekonzentration in der Fermentationsbrühe immer zwischen 10 und 30 g/l liegt. Für die Feed-Dosierung werden eine Waage (Firma Satorius, Typ LP6200S), ein Dosiermodul (Firma Satorius, Typ YFC02Z-V2) und eine Pumpe (Firma Meredos, Typ HP60) eingesetzt. Während der Kultivierung wird der Verlauf der optischen Dichte bei einer Messwellenlänge von
600 nm (OD₆₀₀) mit dem Photometer (Firma Pharmacia Biotech, Typ Ultrospec 2000) bestimmt. Die Konzentrationen an Glucose und Succinat im zellfreien Überstand (Filtration der Kulturbrühe mit Spritzen Fa. Braun, Injekt 2mL sowie Spritzenvorsatzfilter Fa. Millipore, Typ Millipore GP; ∅
33mm; 0,22µm Porenweite; PES-Express-Membran) werden mittels HPLC (stationäre Phase: Aminex HPX-87 H, 300 x 7,8 mm [Firma Biorad], mobile Phase: 5 mM H2SO4, RI-Detektion) quantifiziert.
Am Ende der Kultivierung wird die Fermentationsbrühe in 3 I-Erlenmeyerkolben abgelassen und für 20 Minuten bei 121 °C im Autoklav sterilisiert.
Vorzugsweise wird vor der weiteren Verarbeitung die Biomasse abgetrennt, beispielsweise mittels Laborzentrifuge.

Das Vorkulturmedium enthält die in Tabelle 2 angegebenen Bestandteile. Zur Herstellung des Vorkulturmediums werden 22,0 g Glucose-Monohydrat, 10,0 g Yeast Extract, 20,0 g Tryptone, 0,9 g K₂HPO₄x3H₂O, 1,14 g KH₂PO₄, 0,25 g CaCl₂x2H₂O, 3,0 g (NH₄)₂SO₄, 0,5 g MgSO₄x7H₂O, 20 ml einer Biotin-Lösung (50 mg/l, VE-Wasser als Lösungsmittel) und 1 ml einer Thiamin-Lösung (1 g/l, VE-Wasser als Lösungsmittel) 0,95 Liter vollentsalztem (VE-) Wasser zugesetzt. Unter Rühren wird der pH-Wert mit 2 N NaOH-Lösung auf 7,0 eingestellt, anschließend mit VE-Wasser auf 1,0 Liter aufgefüllt. Die Sterilisation erfolgt durch Sterilfiltration über Stericups mit Millipore Express PLUS Membran mit 0,22 µm Porenweite (Fima Millipore).

**Tabelle 2: Vorkulturmedium**

| **Medienbestandteil** | **Konzentration** |
|---|---|
| Glucose-Monohydrat | 22,0 g/l |
| Yeast Extract | 10,0 g/l |
| Tryptone | 20,0 g/l |
| K₂HPO₄x3H₂O | 0,9 g/l |
| KH₂PO₄ | 1,14 g/l |
| CaCl₂x2H₂O | 0,25 g/l |
| (NH₄)₂SO₄ | 3,0 g/l |
| MgSO₄x7H₂O | 0,5 g/l |
| Biotin | 1,0 mg/l |
| Thiamin | 1,0 mg/l |

Das Batchmedium für die Hauptkultur enthält die in Tabelle 3 angegebenen Bestandteile. Zur Herstellung des Batchmediums werden 154,0 g Glucose-Monohydrat, 35,0 g Yeast Extract, 70,0 g Tryptone, 3,15 g K₂HPO₄x3H₂O, 3,99 g KH₂PO₄, 0,88 g CaCl₂x2H₂O, 10,5 g (NH₄)₂SO₄, 1,75 g MgSO₄x7H₂O, 70 ml einer Biotin-Lösung (50 mg/l, VE-Wasser als Lösungsmittel) und 3,5 ml einer Thiamin-Lösung (1 g/l, VE-Wasser als Lösungsmittel) 3,30 Liter vollentsalztem (VE-) Wasser zugesetzt. Unter Rühren wird der pH-Wert mit 2 N NaOH-Lösung auf 7,0 eingestellt, anschließend mit VE-Wasser auf 3,5 Liter aufgefüllt. Die Sterilisation erfolgt durch Sterilfiltration über Stericups mit Millipore Express PLUS Membran mit 0,22 µm Porenweite (Fima Millipore).

**Tabelle 3: Batchmedium für die Hauptkultur**

| **Medienbestandteil** | **Konzentration** |
|---|---|
| Glucose-Monohydrat | 44,0 g/l |
| Yeast Extract | 10,0 g/l |
| Tryptone | 20,0 g/l |
| K₂HPO₄x3H₂O | 0,9 g/l |
| KH₂PO₄ | 1,14 g/l |
| CaCl₂x2H₂O | 0,25 g/l |
| (NH₄)₂SO₄ | 3,0 g/l |
| MgSO₄x7H₂O | 0,5 g/l |
| Biotin | 1,0 mg/l |
| Thiamin | 1,0 mg/l |

Für die Herstellung des Feedmediums für die Hauptkultur werden 550 g Glucose-Monohydrat in 1 Liter VE-Wasser gelöst. Die Sterilisation erfolgt durch Sterilfiltration über Stericups mit Millipore Express PLUS Membran mit 0,22 µm Porenweite (Firma Millipore).

### Beispiel 2: Reaktivdestillation von DAS zu Succinimid

2.1 1030 g Fermentationsbrühe mit ca. 13 g/l Diammoniumsuccinat (= DAS) wurden mit 58,5 g ca. 95%igem synthetischem Diammoniumsuccinat aufgestockt. Die Trockenmasse der Fermenterbrühe betrug ca. 7 % [hier ist die Biomasse nicht abgetrennt].
   Die aufgestockte Fermentationsbrühe wurde in einem 2 I-Kolben am Rotationsverdampfer eingeengt. Nach dem Einengen blieb eine Schmelze von ca. 150 ml übrig, die in einen 500 ml Vierhalskolben mit PTFE-Rührer, Thermometer, Heizpilz und Brücke überführt wurde. Bei Normaltemperatur wurde 5 Stunden bei 175°C destilliert, danach wurde die Temperatur auf 250°C Sumpftemperatur gesteigert und dort ca. 45 Minuten gehalten. Danach wurde der Druck auf ca. 25 bar verringert und 26,7 g Produkt bei einer Übergangstemperatur von 172 bis 182°C überdestilliert.
   Das Produkt enthielt 88 % Succinimid. Bernsteinsäure, Monoamid oder Diamid wurden nicht detektiert.
2.2 Zu 1450 g Fermenterbrühe mit ca. 30 g/l DAS wurden ca. 120 g synthetisches DAS mit einer Reinheit von ca. 88 % und 101 g Pluriol P600 zugesetzt.
   Die Fermenterbrühe wurde zusammen mit dem Pluriol am Rotationsverdampfer eingeengt. Es wurden 317 g Destillationsaustrag in einen beheizten Tropftrichter auf einem 500-ml-Vierhalskolben mit Heizpilz, Thermometer und Feststoffdestillationsbrücke umgefüllt. Im Vierhalskolben wurden 100 g Pluriol P600 vorgelegt. Das Pluriol wurde auf 240 bis 250°C vorgeheizt. Die eingeengte Fermenterbrühe wurde in den beheizten Kolben getropft und gleichzeitig unter Vakuum (35 mbar) bei einer allmählich auf 310°C steigenden Sumpftemperatur 23 g Produkt abdestilliert.
   Das Produkt enthielt 64 % Succinimid, ca. 4 % Monoamid und ca. 20 % Bernsteinsäure bzw. DAS.
2.3 Es wurde 684 g 25%iges Ammoniakwasser in einen 2000-ml-Zweihalskolben mit PTFE-Rührer vorgelegt. Dann wurde innerhalb von 10 Minuten bei max. 30°C ca. 600 g Bernsteinsäure zugegeben (Kühlung mit Trockeneis). Es wurde 1 Stunde gerührt bis die gesamte Bernsteinsäure gelöst war. Danach wurde der pH-Wert mit Ammoniakwasser auf pH 7 eingestellt. Es wurde das Minimalmedium zur Reaktionslösung gegeben und dann das Reaktionsgemisch am Rotationsverdampfer eingeengt. Der Rückstand wurde in einen 2000 ml Vierhalskolben mit PTFE-Rührer, Thermometer, Heizpilz und Destillationsbrücke umgefüllt. Die Reaktionsmischung wurde bei Normaldruck allmählich auf eine Sumpftemperatur von 250°C aufgeheizt und zunächst Wasser abdestilliert. Der Ansatz wurde noch eine weitere Stunde bei 250°C gerührt. Dann wurde der Sumpf auf 170°C abgekühlt und Vakuum angelegt. Bei 26 mbar und einer Übergangstemperatur von 179 bis 185°C wurden 486 g Produkt abdestilliert. Das Produkt enthielt 92 % Succinimid. Diamid, Monoamid oder Bernsteinsäure wurden als Nebenprodukte nicht detektiert.

In einer Versuchsvariante wurde die Umsetzung von DAS zum Succinimid in einem Dünnschichtverdampfer durchgeführt. Es wurde eine 10- oder 50 %ige synthetische DAS-Lösung eingesetzt und zweistufig über den Dünnschichtverdampfer gefahren. In der ersten Stufe wurde unter Normaldruck Wasser und Ammoniak abdestilliert. Die teilumgesetzte Schmelze lief in den Sumpf. Danach wurde der Sumpf ca. eine Stunde bei 250 °C zur Vervollständigung der Reaktion von DAS zu Succinimid erhitzt. Im zweiten Schritt wurde der Sumpf über den Dünnschichtverdampfer gefahren und das Succinimid über Kopf abdestilliert. Die Manteltemperatur betrug 230 °C bei einem Druck von ca. 30 mbar. Der Zusatz von Pluriol E600 als Sumpfverdünner hat keinen Einfluss auf das Ergebnis der Succinimiddestillation in der zweiten Stufe.
Die Manteltemperatur für den Dünnschichtverdampfer lag für eine 50 %ige DAS-Lösung bei einer Zulaufgeschwindigkeit von 500 l/h bei 170 °C und für eine 10 %ige Lösung bei einer Zulaufgeschwindigkeit von einem Liter pro Stunde bei 220 °C.

Die beschriebene Reaktion wurde neben synthetischem DAS auch für DAS in Minimalmedium und für einen mit DAS aufgestockten Fermenteraustrag durchgeführt: Für ein besseres Handling wurden die teilumgesetzten Succinimidschmelzen jeweils mit Pluriol E600 gemischt. Die Ergebnisse der Versuche sind in Tab. 4 zusammengefasst.

**Tab. 4: Versuchsergebnisse der Dünnschichtverdampfer -Versuche**

| **Einsatz** | **Gehalt Succinimid [Gew. %]** |
|---|---|
| Synth DAS | 97 |
| Synth DAS + Minimalm. + PI. | 85 |
| Synth DAS + Fermenter + PI. | 87 |

### Beispiel 3: Hydrierung

Der in allen Beispielen verwendete Katalysator war vor seinem Einsatz im Wasserstoffstrom aktiviert worden und hatte vor der Wasserstoffaktivierung folgende Zusammensetzung: 63,4 % CoO, 18,1 % CuO, 6,8 % Mn3O4, 3,1 % MoO3, 0,15 % N2O, 3,3 % H3PO4, der zu 100 % fehlende Rest ist Wasser. Die Katalysatorherstellung ist beispielsweise in DE-A 2 321 101 bzw. DE-A 3 904 083 beschrieben.

### 3.1 Herstellung von Succinimid und dessen Hydrierung

50 g/h einer 15 %igen wässrigen Diammoniumsuccinat-Lösung wurden 10 h lang in einem Dünnschichtverdampfer bei 200°C und Normaldruck in eine Destillatfraktion, die überwiegend Wasser enthielt (ca. 30 g/h) und ein Sumpfprodukt, das überwiegend das Succinat enthielt aufgetrennt. Das Sumpfprodukt wurde als Schmelze mit einer Zulauftemperatur von ca. 120°C wiederum in einem Dünnschichtverdampfer diesmal bei 250°C und 50 mbar umgesetzt (Zulauf ca. 40 g/h). Als Destillat (ca. 13 g/h) wurde überwiegend Succinimid mit geringen Gehalten an Wasser und Ammoniak erhalten. Das Sumpfprodukt enthielt neben Succinimid die Verunreinigungen aus der ursprünglichen Diammoniumsuccinat-Lösung. Insgesamt wurden ca. 65 g Succinimid erhalten, die in einem Autoklaven an 10 g Katalysator in Gegenwart von 40 ml 25%iger wässriger Ammoniaklösung innerhalb von 24 h hydriert wurden. Im Reaktionsaustrag wurde Pyrrolidon mittels quantitativer gaschromatographischer Analyse mit 95%iger Ausbeute gefunden. Daneben lagen noch ca. 2 % Succinimid, < 0,1 % gamma Butyrolacton, 1 % Pyrrolidin sowie eine Vielzahl, mengenmäßig unbedeutsamer Produkte vor.

### 3.2 Umsetzung von Pyrrolidon zu NMP

In einem von außen beheizbarem Reaktor wurden an 55 g saurem Aluminiumoxid ca. 20 g/h einer 1 : 1 Mischung aus Pyrrolidon und Methanol (Verdampfung an einer 20 ml Glasringschüttung vor dem Katalysator) bei 300°C kontin. umgesetzt. Im Reaktionsaustrag wurden 48 % unumgesetztes Pyrrolidon und 23,3 % Methanol sowie 27 % NMP gefunden.

### 3.3 Umsetzung von Pyrrolidon zu NMP

Analog Beispiel 3.2 wurde statt saurem Aluminiumoxid, 56 g eines Katalysators bestehend aus 80 % Al₂O₃/20 % SiO₂ verwendet. Bei 300°C wurden im Austrag 48,1 % Pyrrolidon, 34,2 % Methanol sowie 16,2 % NMP gefunden. Nach Temperaturerhöhung auf 350°C fanden sich im Austrag 18 % Pyrrolidon, 17 % Methanol sowie 61,3 % NMP.

## Patentansprüche

1. Verfahren zur Herstellung einer Verbindung II oder einer Zusammensetzung enthaltend die Verbindung II, 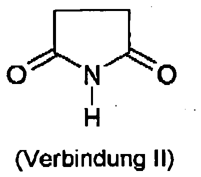 wobei das Verfahren mindestens folgendende Schritte umfasst:
(a) Zurverfügungstellen einer Fermentationsbrühe enthaltend die Verbindung I 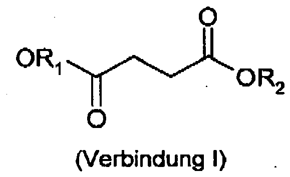 wobei R₁ gleich NH₄⁺, H oder ein anderes Kation und
R₂ gleich NH₄⁺, H oder ein anderes Kation sein kann; und
wobei mindestes R₁ oder R₂ gleich NH₄⁺ ist;
(b) Umsetzen der Verbindung I in die Fermentationsbrühe zur Verbindung II
(c) gleichzeitiges, intermittierendes oder folgendes Abdestillieren von Ammoniak und/oder Wasser;
(d) nachfolgendes Destillieren des Sumpfes der Destillation von Schritt (c) bei vermindertem Druck, wobei ein Destillat enthaltend Verbindung II entsteht;
(e) Isolatieren der Verbindung II oder Umsetzen des Destillats aus Schritt (d).

2. Verfahren nach Anspruch 2, wobei die Verbindung II erhalten wird in einer Zusammensetzung enthaltend weiterhin mindestens 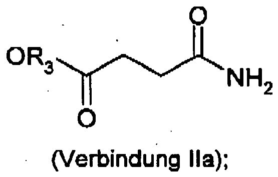 wobei R₃ gleich H oder ein anderes Kation sein kann, und/oder

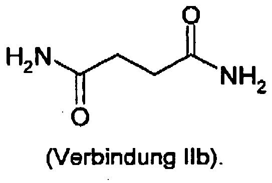

3. Verfahren zur Herstellung einer Verbindung IIIa oder einer Zusammensetzung enthaltend die Verbindung IIIa 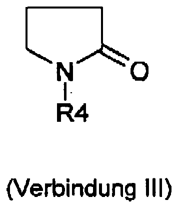 wobei für Verbindung IIIa R₄ gleich H ist;
und wobei das Verfahren die Schritte des Verfahrens nach Anspruch 1 oder 2 enthält und weiterhin folgend:
(f) Reduzieren der Verbindung II zur Verbindung IIIa.

4. Verfahren zur Herstellung einer Verbindung IIIb oder einer Zusammensetzung enthaltend die Verbindung IIIa und IIIb 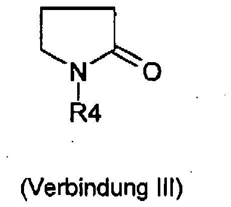 wobei für Verbindung IIIa R₄ gleich H ist; und
wobei für Verbindung IIIb R₄ gleich verzweigtes oder unverzweigtes Alkyl mit 1 bis 20 Kohlenstoffresten mit 0 bis 1 OH- oder NH₂-Resten ist;
und wobei das Verfahren die Schritte des Verfahrens nach Anspruch 3 enthält und weiterhin folgend:
(g) vollständiges oder partielles Alkylieren der Verbindung IIIa zur Verbindung IIIb während oder nach Schritt (f).

5. Verfahren nach Anspruch 4, wobei Pyrrolidon, Hydroxyethylpyrrolidon und/oder N-Methylpyrrolidon (NMP) hergestellt wird.

6. Verfahren nach einem der Ansprüche 1 bis 5, wobei die Umsetzung und Destillation in einem kontinuierlichen Verfahren bei folgenden Bedingungen durchgeführt wird:
(i) optional, Abtrennen der Biomasse und/oder Aufreinigen der Fermentationsbrühe;
(ii) optional, Aufkonzentieren der Fermentationsbrühe;
(iii) Abdestillieren der Nebenprodukte und Umsetzen der Verbindung I bei einer Temperatur von 100 bis 300°C;
(iv) optional, Nachreagieren bei 150 - ca. 300 °C; und
(v) Abdestillation der Verbindung II oder der Verbindung II und der Verbindung IIa und/oder IIb bei einer Temperatur von 150 °C bis 300 °C und bei Normaldruck oder bei einem Druck von ungefähr 0,01 bis ungefähr 1000 mbar.

7. Verfahren nach einem der Ansprüche 1 bis 5, wobei die Umsetzung und Destillation batch-weise bei folgenden Bedingungen durchgeführt wird:
(i) optional, Abtrennen der Biomasse und/oder Aufreinigen der Fermentationsbrühe;
(ii) optional, Aufkonzentieren der Fermentationsbrühe;
(iii) Abdestillieren der Nebenprodukte und Umsetzen der Verbindung I bei einer Temperatur von RT bis 300°C;
(iv) optional, Nachreagieren des Destillats bei 150 °C bis zu 300°C für weniger als 2h; und
(v) optional, Überdestillation des Reaktionsgemisches bei Normaldruck oder verringertem Druck und bei 150 °C bis 300 °C.

8. Verfahren nach einem der Ansprüche 1 bis 7, wobei die Fermentationsbrühe vor der Reaktion der Verbindung I oder vor Destillation der Verbindung II bis zu 50% Sumpfverdünner zugegeben wird.

9. Verfahren nach einem der Ansprüche 4 bis 7, wobei das Reduzieren der Verbindung II zur Verbindung III eine Hydrierung ist.

10. Verfahren nach einem der Ansprüche 4 bis 8, wobei das Reduzieren eine Gas- oder Flüssigphasenhydrierung ist, die bei folgenden Bedingungen durchgeführt wird:
(i) bei einer Temperatur zwischen 80°C und 330°C;
(ii) mit einem homogenen oder heterogenen Katalysator geeignet zur Hydrierung von Carbonylgruppen, der ein oder mehrere Elemente der Gruppe 11, 6, 7, 8-10, 13, 14 oder 15 des Periodensystems der Elemente enthält; und/oder
(iii) in der Gasphase bei einem Druck zwischen Normaldruck und 130bar oder in der Flüssigphase bei einem Druck zwischen 20 und 400bar.

11. Verfahren nach einem der Ansprüche 4 bis 10, wobei das Verfahren folgenden Schritt enthält:
Alkylieren der Verbindung IIIa mit einem verzweigten oder unverzweigten gesättiten Kohlenwasserstoff mit 1 bis 20 Kohlenstoffresten und 1 bis 2 OH- oder NH₂-Resten.

12. Verfahren nach Anspruch 11, wobei das Alkylieren in der Gasphase bei folgenden Bedingungen durchgeführt wird:
(i) bei einer Temperatur von 50 bis 600°C,;
(ii) bei einem Druck von Normaldruck bis 100bar;
(iii) Einsetzen der Alkylierungskomponente stöchiometrisch oder überstöchiometrisch,; und
(iv) Verwenden eines basischen, neutralen oder lewis- bzw. brönstedsauren Oxids oder Mischoxid oder gemischten Oxids oder eines Metalls.

13. Verfahren nach Anspruch 11, wobei die Alkylierung in der flüssigen Phase bei folgenden Bedingungen durchgeführt wird:
(i) bei einer Temperatur von 50 bis 600°C;
(ii) bei einem Druck von Normaldruck bis 325bar;
(iii) Einsetzen der Alkylierungskomponente stöchiometrisch oder überstöchiometrisch; und
(iv) Verwenden eines basischen, neutralen oder lewis- bzw. brönstedsauren Oxids oder Mischoxid oder gemischten Oxids oder eines Metalls.

14. Zusammensetzung enthaltend Succinimid und Polyalkylenglykole, Silikonöle oder Paraffinöle.

## Claims

1. A process for preparing a compound II or a composition comprising said compound II 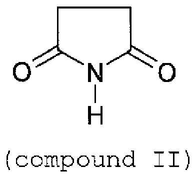 the process comprising at least the following steps:
(a) providing a fermentation broth comprising a compound I 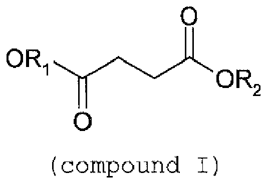 where R₁ may be NH₄⁺, H or another cation and
R₂ may be NH₄⁺, H or another cation; and
where at least R₁ or R₂ is NH₄⁺;
(b) converting said compound I in said fermentation broth to said compound II;
(c) simultaneously, intermittently or subsequently distillatively removing ammonia and/or water;
(d) subsequently distilling the bottoms of the distillation of step (c) under reduced pressure to form a distillate comprising said compound II;
(e) isolating said compound II or converting said distillate from step (d).

2. The process according to claim 1, wherein said compound II is obtained in a composition further comprising at least 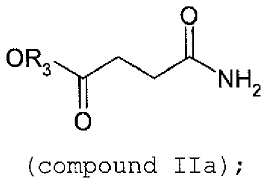 where R₃ may be H or another cation
and/or

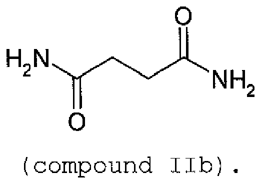

3. A process for preparing a compound IIIa or a composition comprising said compound IIIa 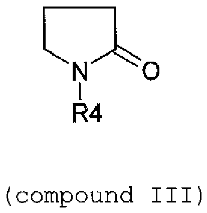 where R₄ for compound IIIa is H; and where the process comprises the steps of the process according to claim 1 or 2, followed by:
(f) reducing said compound II to said compound IIIa.

4. A process for preparing a compound IIIb or a composition comprising said compounds IIIa and IIIb 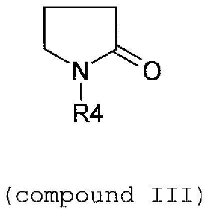 where R₄ for said compound IIIa is H; and
where R₄ for said compound IIIb is branched or unbranched alkyl having from 1 to 20 carbon residues with from 0 to 1 OH or NH₂ radicals;
and where the process comprises the steps of the process according to claim 3, followed by:
(g) completely or partially alkylating said compound IIIa to give said compound IIIb during or after step (f).

5. The process according to claim 4, wherein pyrrolidone, hydroxyethylpyrrolidone and/or N-methylpyrrolidone (NMP) is prepared.

6. The process according to any of claims 1 to 5, wherein said converting and distillation are performed in a continuous process under the following conditions:
(i) optionally, removing the biomass and/or purifying said fermentation broth;
(ii) optionally, concentrating said fermentation broth;
(iii) distillatively removing the by-products and converting said compound I at a temperature of from 100 to 300°C;
(iv) optionally, postreacting at 150 - approx. 300°C; and
(v) distillatively removing said compound II or said compound II and said compound IIa and/or IIb at a temperature of from 150°C to 300°C and at standard pressure or at a pressure of from about 0.01 to about 1000 mbar.

7. The process according to any of claims 1 to 5, wherein said converting and distillation are performed batchwise under the following conditions:
(i) optionally, removing the biomass and/or purifying said fermentation broth;
(ii) optionally, concentrating said fermentation broth;
(iii) distillatively removing the by-products and converting said compound I at a temperature of from RT to 300°C;
(iv) optionally, postreacting the distillate at from 150°C up to 300°C for less than 2 h; and
(v) optionally, overdistillating the reaction mixture at standard pressure or reduced pressure and at from 150°C to 300°C.

8. The process according to any of claims 1 to 7, wherein said fermentation broth, before the reaction of said compound I or before distillation of said compound II, has up to 50% of a bottoms diluent added to it.

9. The process according to any of claims 4 to 7, wherein said reducing of said compound II to said compound III is a hydrogenation.

10. The process according to any of claims 4 to 8, wherein said reducing is a gas phase or liquid phase hydrogenation which is performed under the following conditions:
(i) at a temperature between 80°C and 330°C;
(ii) with a homogeneous or heterogeneous catalyst suitable for the hydrogenation of carbonyl groups which comprises one or more elements of group 11, 6, 7, 8-10, 13, 14 or 15 of the Periodic Table of the Elements; and/or
(iii) in the gas phase at a pressure between standard pressure and 130 bar or in the liquid phase at a pressure between 20 and 400 bar.

11. The process according to any of claims 4 to 10, which comprises the following step:
alkylating said compound IIIa with a branched or unbranched, saturated hydrocarbon having from 1 to 20 carbon residues and from 1 to 2 OH or NH₂ radicals.

12. The process according to claim 11, wherein said alkylating is performed in the gas phase under the following conditions:
(i) at a temperature of from 50 to 600°C;
(ii) at a pressure of from standard pressure to 100 bar;
(iii) using the alkylating component in a stoichiometric or superstoichiometric amount; and
(iv) using a basic, neutral or Lewis- or Brønsted-acidic oxide or interoxide or mixed oxide or a metal.

13. The process according to claim 11, wherein said alkylating is performed in the liquid phase under the following conditions:
(i) at a temperature of from 50 to 600°C;
(ii) at a pressure of from standard pressure to 325 bar;
(iii) using the alkylating component in a stoichiometric or superstoichiometric amount; and
(iv) using a basic, neutral or Lewis- or Brønsted-acidic oxide or interoxide or mixed oxide or a metal.

14. A composition comprising succinimide and polyalkylene glycols, silicone oils or paraffin oils.

## Revendications

1. Procédé de fabrication d'un composé II ou d'une composition contenant le composé II 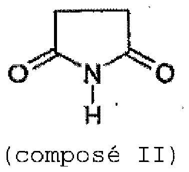 le procédé comprenant au moins les étapes suivantes :
(a) la mise à disposition d'un bouillon de fermentation contenant le composé I 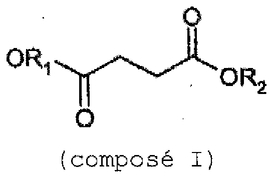 R₁ pouvant représenter NH₄⁺, H ou un autre cation, et
R₂ pouvant représenter NH₄⁺, H ou un autre cation ; et
au moins R₁ ou R₂ représentant NH₄⁺;
(b) la transformation du composé I dans le bouillon de fermentation en composé II ;
(c) l'élimination par distillation simultanée, intermittente ou ultérieure de l'ammoniac et/ou de l'eau ;
(d) la distillation ultérieure du fond de la distillation de l'étape (c) à pression réduite, un distillat contenant le composé II se formant ;
(e) l'isolation du composé II ou la transformation du distillat de l'étape (d).

2. Procédé selon la revendication 1, dans lequel le composé II est obtenu dans une composition contenant également au moins 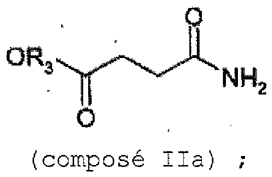 R₃ pouvant représenter H ou un autre cation,
et/ou

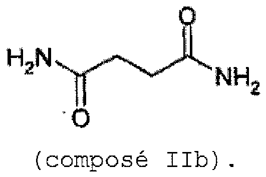

3. Procédé de fabrication d'un composé IIIa ou d'une composition contenant le composé IIIa 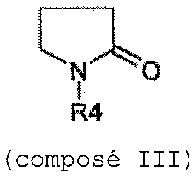 R₄ représentant H pour le composé IIIa ;
et le procédé contenant les étapes du procédé selon la revendication 1 ou 2, et également l'étape suivante : (f) la réduction du composé II en composé IIIa.

4. Procédé de fabrication d'un composé IIIb ou d'une composition contenant le composé IIIa et IIIb 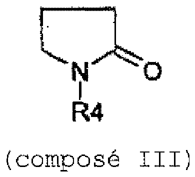 R₄ représentant H pour le composé IIIa ; et
R₄ représentant un alkyle ramifié ou non ramifié contenant 1 à 20 radicaux carbone et 0 à 1 radical OH ou NH₂ pour le composé IIIb ;
et le procédé contenant les étapes du procédé selon la revendication 3, et également l'étape suivante :
(g) l'alkylation totale ou partielle du composé IIIa en composé IIIb pendant ou après l'étape (f).

5. Procédé selon la revendication 4, dans lequel la pyrrolidone, l'hydroxyéthylpyrrolidone et/ou la N-méthylpyrrolidone (NMP) sont fabriquées.

6. Procédé selon l'une quelconque des revendications 1 à 5, dans lequel la réaction et la distillation sont réalisées en un procédé continu dans les conditions suivantes :
(i) éventuellement la séparation de la biomasse et/ou la purification du bouillon de fermentation ;
(ii) éventuellement la concentration du bouillon de fermentation ;
(iii) l'élimination par distillation des produits secondaires et la mise en réaction du composé I à une température de 100 à 300°C;
(iv) éventuellement la post-réaction à une température de 150 à environ 300 °C ; et
(v) l'élimination par distillation du composé II ou du composé II et du composé IIa et/ou IIb à une température de 150°C à 300 °C et à pression normale ou à une pression d'environ 0,01 à environ 1 000 mbar.

7. Procédé selon l'une quelconque des revendications 1 à 5, dans lequel la réaction et la distillation sont réalisées de manière discontinue dans les conditions suivantes :
(i) éventuellement la séparation de la biomasse et/ou la purification du bouillon de fermentation ;
(ii) éventuellement la concentration du bouillon de fermentation ;
(iii) l'élimination par distillation des produits secondaires et la mise en réaction du composé I à une température de la TA à 300°C;
(iv) éventuellement la post-réaction du distillat à une température de 150 °C à 300 °C pendant moins de 2 h ; et
(v) éventuellement la sur-distillation du mélange réactionnel à pression normale ou à pression réduite et à une température de 150 °C à 300 °C.

8. Procédé selon l'une quelconque des revendications 1 à 7, dans lequel jusqu'à 50 % de diluant de fond est ajouté au bouillon de fermentation avant la mise en réaction du composé I ou avant la distillation du composé II.

9. Procédé selon l'une quelconque des revendications 4 à 7, dans lequel la réduction du composé II en composé III est une hydrogénation.

10. Procédé selon l'une quelconque des revendications 4 à 8, dans lequel la réduction est une hydrogénation en phase gazeuse ou liquide, qui est réalisée dans les conditions suivantes :
(i) à une température comprise entre 80 °C et 330°C ;
(ii) avec un catalyseur homogène ou hétérogène approprié pour l'hydrogénation de groupes carbonyle, qui contient un ou plusieurs éléments du groupe 11, 6, 7, 8 à 10, 13, 14 ou 15 du tableau périodique des éléments ; et/ou
(iii) en phase gazeuse à une pression comprise entre la pression normale et 130 bar ou en phase liquide à une pression comprise entre 20 et 400 bar.

11. Procédé selon l'une quelconque des revendications 4 à 10, dans lequel le procédé contient l'étape suivante :
l'alkylation du composé IIIa avec un hydrocarbure saturé ramifié ou non ramifié contenant 1 à 20 radicaux carbone et 1 à 2 radicaux OH ou NH₂.

12. Procédé selon la revendication 11, dans lequel l'alkylation est réalisée en phase gazeuse dans les conditions suivantes :
(i) à une température de 50 à 600 °C ;
(ii) à une pression de la pression normale à 100 bar ;
(iii) utilisation stoechiométrique ou surstoechiométrique du composant d'alkylation ; et
(iv) utilisation d'un oxyde ou oxyde mixte ou oxyde composite basique, neutre ou acide de Lewis ou de Brönsted ou d'un métal.

13. Procédé selon la revendication 11, dans lequel l'alkylation est réalisée en phase liquide dans les conditions suivantes :
(i) à une température de 50 à 600°C;
(ii) à une pression de la pression normale à 325 bar ;
(iii) utilisation stoechiométrique ou surstoechiométrique du composant d'alkylation ; et
(iv) utilisation d'un oxyde ou oxyde mixte ou oxyde composite basique, neutre ou acide de Lewis ou de Brönsted ou d'un métal.

14. Composition contenant du succinimide et des polyalkylène glycols, des huiles de silicone ou des huiles de paraffine.
